# EUROPEAN PATENT APPLICATION

(11) **EP 2 230 615 A1**
(43) Date of publication of application: **22.09.2010**
(21) Application number: 09166185.0
(22) Date of filing: 23.07.2009
(51) Int. Cl.: G06F 19/00

(54) **Apparatus for handling medical therapies of patients in a sanitary facility, particularly for the management and monitoring of medical therapies**

(30) Priority: 20.03.2009 IT MI20090433
(71) Applicant: Unihospital S.r.l., 38040 Ravina (TN) (IT)
(72) Inventor: Bertoldi, Paolo, 38040, Ravina TN (IT)
(74) Representative: Modiano, Micaela Nadia

(57) **Abstract**

An apparatus (1) for handling medical therapies of patients in a sanitary facility, particularly for the management and monitoring of medical therapies, comprising first control means (2) and second control means (3), which are functionally connected to drug data banks, the first control means (2) and the second control means (3) being usable respectively by medical staff to prescribe medical therapies that the patient must undergo and by nursing staff to monitor the medical therapies, the first control means (2) comprising first means for reading a unique code (6) for the identification of the patient and the second control means (3) comprising second means for reading the unique code (6) and first and second codes (10, 11) of the drug (17) to be administered to the patient.

## Description

The present invention relates to an apparatus for handling medical therapies of patients in a sanitary facility, particularly for the management and monitoring of medical therapies.

As is known, the management and monitoring of medical therapies prescribed to patients use apparatuses that allow medical staff and nursing staff to identify correctly and uniquely the patient so as to be able to administer to such patient the correct drugs with the correct dosages, at the correct time and according to the correct administration method.

To this end, patient identification devices are known which consist in handheld computers, which are capable of reading a code that identifies the patient uniquely and is typically applied to an identification wristband that can be worn by the patient, and are functionally connected to computerized data banks which collect a plurality of items of information regarding the several applicable therapies, including information related to the drugs that can be administered.

In this manner, medical staff is able to prescribe the medical therapy that is most suited for the patient directly in the patient's hospital room, having available on the handheld computer all the information described above.

The monitoring and the functional application of the therapy prescribed by the medical staff are entrusted to the nursing staff, which also have at their disposal handheld computers for performing in detail the prescribed therapy after identifying uniquely the patient again by means of the identification wristband.

These known types of apparatus are not devoid of drawbacks, which include the fact that the exchange of data and/or information among the devices with which the medical staff is equipped and the devices with which the nursing staff is equipped and the data banks is not always reliable, since the occurrence of inconsistencies between the drugs used and the prescribed medical therapy is not infrequent.

The aim of the present invention is to provide an apparatus for the handling of medical therapies of patients in a sanitary facility, particularly for the management and monitoring of medical therapies, which allows to manage and monitor the implementation of the therapy, allowing medical staff to prescribe medical therapies that can be managed and applied easily by the nursing staff.

Within this aim, an object of the present invention is to allow correct identification of the patient by medical staff and nursing staff.

This aim, as well as these and other objects that will become better apparent hereinafter, are achieved by an apparatus for the handling of medical therapies of patients in a sanitary facility, particularly for the management and monitoring of medical therapies, comprising first control means and second control means which communicate functionally with drug data banks and can be used respectively by medical staff to prescribe medical therapies to which the patient is to be subjected and by nursing staff to monitor said medical therapies, said first control means comprising first means for reading a unique identification code of said patient and said second control means comprising second means for reading said unique code and a first code and a second code of the drug to be administered to said patient.

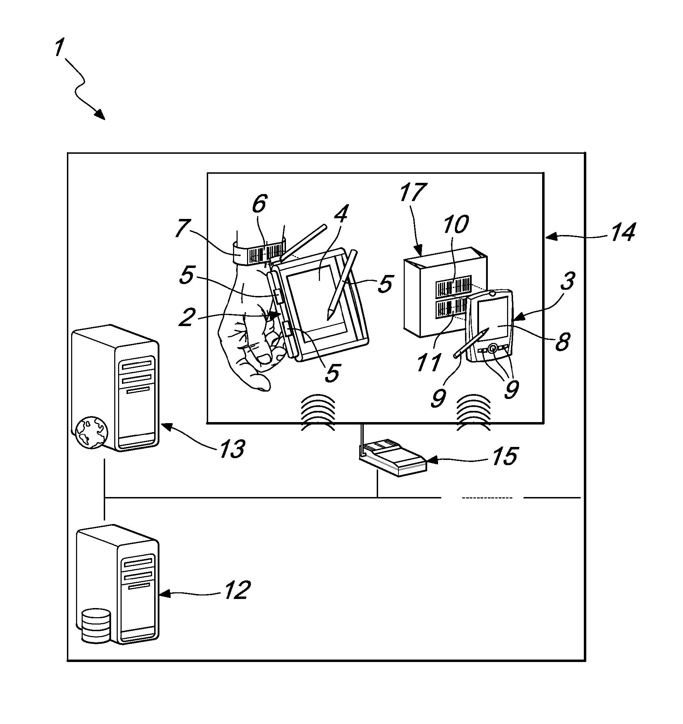

Further characteristics and advantages of the present invention will become apparent from the description of a preferred but not exclusive embodiment of an apparatus for the handling of medical therapies of patients in a sanitary facility, particularly for the management and monitoring of medical therapies, according to the invention, illustrated by way of non-limiting example in the accompanying drawing, wherein the sole Figure illustrates a schematic representation of the apparatus according to the invention.

With reference to the Figure, the apparatus for the handling of medical therapies of patients in a sanitary facility, particularly for the management and monitoring of medical therapies, generally designated by the reference numeral 1, uses identification technologies based on barcodes or radio frequencies for the authentication of the patient to whom the therapy is to be administered and to check such therapy.

Moreover, as described in greater detail hereinafter, the apparatus 1 uses a drug data bank enhanced with information indispensable to the medical staff and to the nursing staff for the correct prescription and administration of the therapy.

More particularly, the apparatus 1 comprises first control means 2 and second control means 3, which communicate functionally with drug data banks and can be used respectively by medical staff to prescribe medical therapies to which the patient is to be subjected and by nursing staff to monitor the medical therapies.

The first control means 2, which can consist for example of a first portable or handheld computer provided with a first graphical interface 4 and with first means 5 for the entry and/or selection of data for prescribing medical therapies, comprise first means for reading a unique patient identification code 6, which is associated typically with an identification wristband 7 that can be worn by the patient.

More particularly, the unique code 6 can be printed on the identification wristband 7, for example in the form of a barcode, or can consist of a radio signal emitted by an electronic radio frequency circuit (RFID) installed directly in the identification wristband 7.

Depending on whether working with a barcode or with a radio frequency signal is selected, the first reading means can comprise respectively first optical readers for reading the unique code 6 that is printed on the identification wristband 7 or can comprise first radio frequency receivers to receive the radio signal emitted by the electronic radio frequency circuit installed in the identification wristband 7.

As regards the second control means 3, which can consist for example of a second portable or handheld computer provided with a first graphical interface 4 and with first means 5 for entering and/or selecting data for monitoring the medical therapies, they comprise second means for reading the unique code 6 and a first code 10 and a second code 11 of the drug 17 to be administered to the patient.

More precisely, the first and second codes 10 and 11 consist of a first barcode 10, which identifies the drug 17 and is issued by the Health Ministry, and of a second barcode 11 of a serial type, which identifies the specific package.

Depending on whether working with a unique code 6 that consists of a barcode or of a radio frequency signal is selected, the second reading means can comprise second optical readers for reading the unique code 6 printed on the identification wristband 7 and for reading the double code 10 and 11 of the drug 17, or can comprise second radio frequency receivers for receiving the radio signal emitted by the electronic radio frequency circuit installed in the identification wristband 7 and second optical readers for reading the double code 10 and 11 of the drug 17.

Advantageously, in order to ensure privacy of the patient and prevent the intervention of unauthorized individuals in the management of the medical therapy, means for identifying medical staff and nursing staff are provided which are associated respectively with the first portable computer and with the second portable computer.

More precisely, both medical staff and nursing staff can be authenticated to a system, known as Safe Administration System (hereinafter SAS), by means of an access with login and password or by means of an identification card, also provided with an electronic radio frequency circuit. In this manner, the patient, the medical staff, the nursing staff, the prescription of the medical therapy and the administration thereof are inextricably tied to each other and are consequently stored in the filing systems of the SAS system.

As shown in the Figure, such filing systems of the SAS system can consist of one or more servers 12 and 13 which are functionally connected to the internal network of the sanitary facility and/or to the external Internet.

Advantageously, the server 12 functionally connected to the internal network of the sanitary facility contains the data banks related to the drugs enhanced with properties, characteristics and information related to patients and to the essential therapies both for medical staff and for nursing staff.

In order to ensure the most complete mobility of medical staff and nursing staff in performing the operations for prescribing and administering medical therapies directly at the patient's bed in the hospital room 14, both the first portable computer and the second portable computer comprise transceiver means which communicate functionally with at least one transceiver station 15 associated with the SAS system, i.e., with the data banks, for the wireless exchange of data and/or information between the transceiver station 15 and the portable computers used by the medical and nursing staff.

Operation of the apparatus 1 for the handling of medical therapies of patients in a sanitary facility, particularly for the management and monitoring of medical therapies, is described hereinafter.

Functionally, the SAS system verifies and logs the administration of the correct therapy to the correct patient with the correct dosage by means of the correct administration path and at the correct time.

A particularly advantageous functionality of the SAS system is constituted by the possibility to manage the entire flow of prescription by means of a multilanguage voice interface, with speaker-independent continuous speech recognition, which simplifies and speeds up the entry, modification and suspension of therapies.

The process is managed by means of suitable software modules that communicate with the data banks that contain the information related to the patients, such as the therapy prescribed by medical staff, any allergies, the status and condition of the patient, and the history of administrations performed.

The SAS system compares the identity of the patient with the therapy and checks that the therapy is correct for the specific patient.

Tracing with the unique code 6, as a barcode or RFID electronic circuit, and with the double code 10 and 11 of the drugs to be administered, ensures complete and accurate filing of the handling of the patient throughout the stay in the sanitary facility.

In practice it has been found that the apparatus for the handling of medical therapies of patients in a sanitary facility, particularly for the management and monitoring of medical therapies, according to the present invention, fully achieves the intended aim and objects, since it allows to manage and monitor the execution of the therapy, allowing medical staff to prescribe medical therapies that are tailored to each individual patient and can be easily managed and applied by nursing staff by guiding them to the correct dosage of the drug, at the correct time and with the correct method of administration.

More particularly, the apparatus according to the present invention, which allows the introduction of a type of prescription according to a parameter-based scheme, in which the dosage to be administered is adjusted by a vital parameter of the patient, allows the management of the prescription according to a weekly scheme, an insulin scheme, the management of the aggregation of two or more drugs both during prescription and during administration, the management of preparations with generation of a unique code, the management of time-based therapies (with particular reference to the total infused volume), the management of the prescription of bolus therapies (i.e., the administration of a drug as a massive dose that is not split over time), the management of the contexts and properties on the part of the central system, the management of the inheritance of such contexts, the management of the division of visiting rounds and of the administration for each individual ward, the management of the prescription flow of therapies with confirmation by the physician, the management of non-replaceable products, the management of prescription notes with validity periods, the management of a further level of detail for visualizing the therapy of a day and the entry of activities for the management of bandage removal and the rate change of infusion therapies, and automatic continuation of the therapy.

Another advantage of the apparatus according to the present invention consists in that it warns the medical staff and the nursing staff of the expiration of schemes that have a termination date and of the verification that the administration session has been closed.

Another advantage of the apparatus according to the present invention consists in that it can be used "autonomously" for the management of home therapy with the subsequent possibility to synchronize the data when the handheld computer returns to base.

Another advantage of the apparatus according to the present invention consists in that during the therapy administration step the identification of the drug with reading of the double code of the drug, besides ensuring a consistency check with respect to the prescribed drug, allows to trace the information related to the package of the drug that is used.

This last information contains the exact data item of the consumption of drugs and allows the administration of the medical facility to reduce considerably the management costs of drug logistics.

The apparatus for the handling of medical therapies of patients in a sanitary facility, particularly for the management and monitoring of medical therapies, thus conceived is susceptible of numerous modifications and variations, all of which are within the scope of the appended claims.

All the details may further be replaced with other technically equivalent elements.

In practice, the materials used, so long as they are compatible with the specific use, as well as the contingent shapes and dimensions, may be any according to requirements and to the state of the art.

The disclosures in Italian Patent Application no. MI2009A000433, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An apparatus for handling medical therapies of patients in a sanitary facility, particularly for the management and monitoring of medical therapies, comprising first control means (2) and second control means (3), which are functionally connected to data banks of drugs and can be used respectively by medical staff to prescribe medical therapies that the patient must undergo and by nursing staff to monitor said medical therapies, said first control means (2) comprising first means for reading a unique code (6) for the identification of said patient and said second control means (3) comprising second means for reading said unique code (6) and first and second codes (10, 11) of the drug (17) to be administered to said patient.

2. The apparatus according to claim 1, **characterized in that** said first control means (2) comprise a first portable or handheld computer that is provided with a first graphical interface (4) and with first means (5) for the entry and/or selection of data for the prescription of said medical therapies.

3. The apparatus according to one or more of the preceding claims, **characterized in that** it comprises an identification wristband (7) that can be worn by said patient and is associated with said unique code (6).

4. The apparatus according to one or more of the preceding claims, **characterized in that** said unique code (6) is printed on said identification wristband (7) and **in that** said first reading means comprise first optical readers for reading said unique printed code (6).

5. The apparatus according to one or more of the preceding claims 1 to 3, **characterized in that** said unique code (6) consists of a radio signal that is emitted by an electronic radio frequency circuit installed in said identification wristband (7), and **in that** said first reading means comprise first radio frequency receivers for receiving the radio signal emitted by said electronic radio frequency circuit.

6. The apparatus according to one or more of the preceding claims 4 or 5, **characterized in that** said second control means (3) comprise a second portable or handheld computer provided with a second graphical interface (8) and with second means (9) for the entry and/or selection of data for monitoring said medical therapies.

7. The apparatus according to claim 6, **characterized in that** said second reading means comprise second optical readers for reading said unique printed code (6) and for reading said first and second codes (10, 11) of the drug (17), said first drug code being a drug identification code, said second drug code being a code of the serial type that identifies the specific package of said drug.

8. The apparatus according to claim 6, **characterized in that** said second reading means comprise second radio frequency receivers for receiving the radio signal emitted by said electronic radio frequency circuit and second optical readers for reading said double code (10, 11) of the drug (17).

9. The apparatus according to one of the preceding claims 7 or 8, **characterized in that** said first portable computer and said second portable computer comprise transceiver means which are functionally connected to at least one transceiver station (15), which is associated with said data banks for the wireless exchange of data and/or information between said transceiver station (15) and said first portable computer and said second portable computer.

10. The apparatus according to one or more of the preceding claims, **characterized in that** it comprises means for identifying said medical staff and said nursing staff associated respectively with said first portable computer and with said second portable computer.
